# EUROPEAN PATENT SPECIFICATION

(11) **EP 0 791 363 B1**
(45) Date of publication and mention of the grant of the patent: **23.04.2003**
(21) Application number: 97102566.3
(22) Date of filing: 18.02.1997
(51) Int. Cl.: A61L 2/20, A61L 2/26, A61L 11/00, B02C 19/12

(54) **Disposable apparatus for the production and the instantaneous delivery of disinfectant gas**
Einwegvorrichtung zur Produktion und Sofortabgabe von desinfektierenden Gasen
Appareil à usage unique pour la production et la livraison instantanée de gaz désinfectant

(30) Priority: 21.02.1996 IT MI960128 U
(43) Date of publication of application: 27.08.1997
(73) Proprietor: Eurochem Research S.r.l., 95127 Catania (IT)
(72) Inventor: Sollima, Francesco, 95127 Catania (IT); Sollima, Cristiano, 95127 Catania (IT)
(74) Representative: Gervasi, Gemma, Dr.

(56) References cited:
- EP-A- 0 336 047
- WO-A-84/04036
- GB-A- 1 448 337
- US-A- 5 118 471

## Description

The present invention refers to a disposable apparatus for the production and the instantaneous delivery of disinfectant gas showing remarkable practicality and utility characteristics towards the means used for the same purpose.

It is known that an important problem in the field of the disposal of solid wastes consists of the solid materials coming from hospitals, biological laboratories and such.

These materials can not be directly sent to the incineration but they must previously undergo disinfection.

At present the disinfection is carried out by treatment with liquid disinfectants but this treatment shows various drawbacks.

First of all this treatment is not very practical from the operative point of view and in addition it is not very efficient as an homogeneous distribution of the liquid disinfectant in the heterogeneous mass of the solid wastes turns out to be impossible.

The apparatus according to the present invention solves in a practical way the problem of the disinfection of the solid wastes already loaded into the bags destined to the incineration and more generally the problem of disinfection of little environments by a disinfectant gas.

### BRIEF DESCRIPTION OF THE FIGURES

The Figure 1 represents the apparatus before the use;
the Figures 2, 3 and 4 show the situation of the apparatus after the use and in particular Figure 2 shows the lid removed from the plug, Figure 3 shows the reaction chamber after use and Figure 4 shows the pin which before use provides for preventing the entrance of air into the reaction chamber.

Referring to the numerical symbols of the various Figures, the apparatus essentially consists of:
- a reaction chamber (1);
- a plug-tank (3);
- a punch (5);
- a lid (12) for said plug tank;
- two truncated pipes (9) curving inwards into said reaction chamber;
- two segments of pipe (6) forming a continuous pipe with said truncated pipes, coming out from said reaction chamber;
- a pin (8) entering airtight into said truncated pipes (9).

The reaction chamber (1) has a spherical shape with flat bottom and it has, in the upper part, a cylindrical neck (2) and in equatorial position two truncated pipes (9) curving inwards into the reaction chamber from two diametrically opposed positions and along the same axis.

The two truncated pipes (9) end in positions relatively distant one from the other.

The plug-tank (3) is applied to the neck (2) and internally it has a cylindrical shape coaxial with said neck and closed at the bottom by a diaphragm (4).

The punch (5) has a tubular structure, it is applied in a coaxial manner with the cylindrical part of the plug tank, it is able to slide inside the part itself and it ends in the lower part with a sharp edge having the shape of a mouthpiece of a flute. In the upper part the punch has a flat base equipped with a hole (14).

The lid (12) is applied to the plug-tank, it encloses the punch and it has in the base an indentation (13) which, before use, closes the hole (14) of the punch.

The segments of pipe (6) are applied coaxially to the truncated pipes (9) and in the part coming out of the reaction chamber they have some holes (11) on the wall and some engravings (7) merlon-shaped at the ends.

The pin (8) has a cylindrical shape and it is equipped at one end with a ring (10) having the function of a handle.

Before using the apparatus the pin (8) is inserted airtight into the two truncated pipes (9) and it prevents the return of the air into the reaction chamber.

In the preferred embodiment the reaction chamber has an inner diameter equal to 7.5-9.5 cm, the neck of the reaction chamber has a height equal to 4.5 cm and the punch has a height equal to 7.5 cm.

The various components of the apparatus are manufactured in a thermoplastic material such as polyethylene, polypropylene or PVC.

The apparatus according to the present invention is particularly suited for the production of chlorine. For this purpose in the plug-tank the solid reagent consisting of dust-shaped halazone is loaded and in the reaction chamber the liquid reagent consisting of an alkaline solution is loaded.

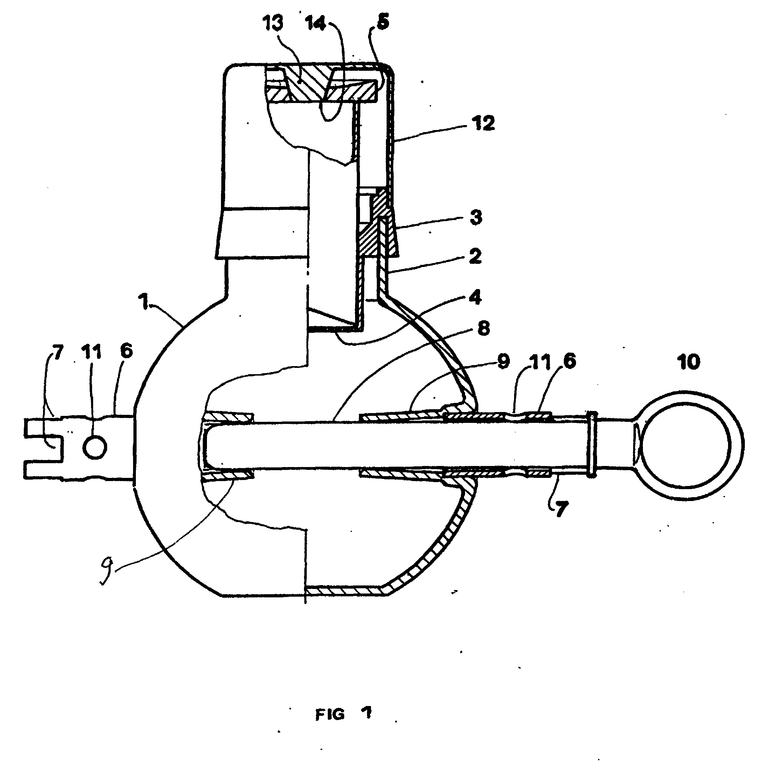

The apparatus, loaded with the two reagents, is stored and transported in the form illustrated in the Figure 1 which guarantees against air and humidity entrance.

At the moment of use the lid (12) and the pin (8) are removed and the punch (5) is pushed which pierces through the diaphragm (4) allowing the fall of the solid reagent and its mixing with the liquid reagent.

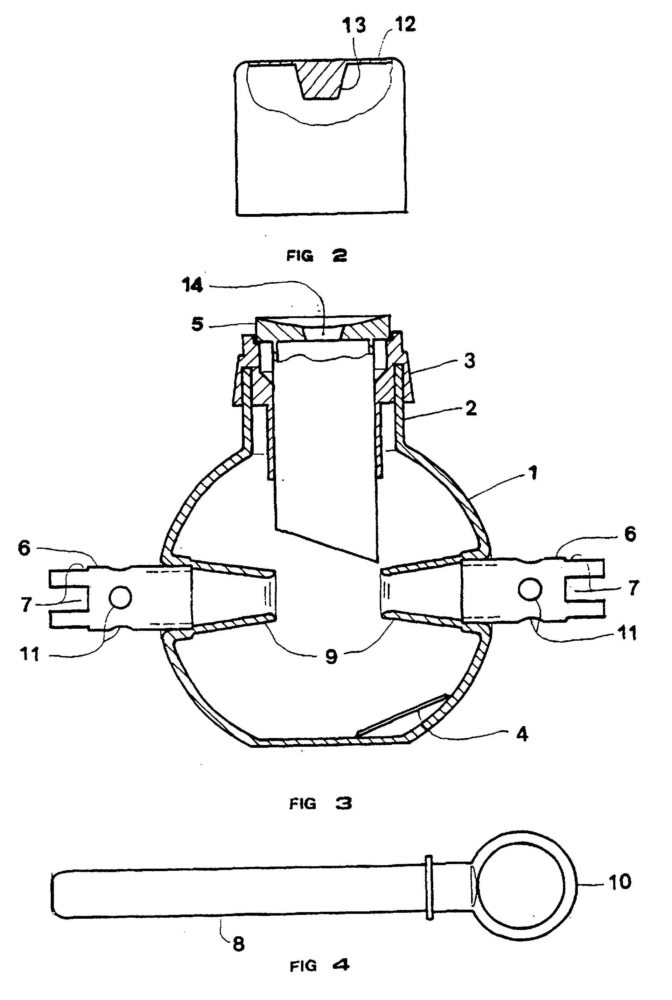

At this moment the apparatus, appearing as illustrated in Figure 3, is introduced in the bag containing the wastes to sterilize which is closed.

From the reaction chlorine at the gaseous state is developed coming out from the holes (14) and (11) and from the engravings (7).

It is proper to point out the following advantageous aspects of the apparatus according to the invention.

First of all the chlorine emission is assured in any disposition which the apparatus takes inside the bag and with any material with which it comes into contact thanks to the numerous and varied openings.

Moreover the emission of the liquid reagent is prevented in any disposition assumed by the apparatus due to the indentations of the truncated pipes (9) and the cylindrical part of the punch (5).

## Claims

1. Disposable apparatus for the production and the instantaneous delivery of disinfectant gas consisting of a reaction chamber (1)
having a spherical shape and a cylindrical neck (2), a plug tank (3) applied to said neck (2) and having a cylindrical shape closed at the bottom by a diaphragm (4), a punch(5) having a tubular structure able to slide inside said plug tank and ending in the lower part with a sharp edge and capable of piercing through said diaphragm (4), a lid (12) for said plug tank, two truncated pipes (9) curving inwards into said reaction chamber and entering the reaction chamber from two diametrically opposed positions and along the same axis and ending in positions relatively distant one from the other, two segments of pipe (6) forming a continuous pipe with said truncated pipes, coming out from said reaction chamber and a pin (8) which is capable of being inserted airtight in the two truncated pipes (9) before the use of the apparatus in order to prevent air from entering into the reaction chamber.

2. Apparatus as claimed in claim 1, **characterized in that** said reaction chamber (1) has a flat bottom and said cylindrical neck (2) is located in the upper part of said reaction chamber (1).

3. Apparatus as claimed in claim 1, **characterized in that** the sharp edge of said punch (5) has the shape of a mouthpiece of a flute.

4. Apparatus as claimed in claim 1, **characterized in that** said punch (5) has a flat base equipped with a hole (14).

5. Apparatus as claimed in claim 1, **characterized in that** said lid (12) has in its base an indentation (13) which, before the use, closes the hole (14) of said punch (5).

6. Apparatus as claimed in claim 1, **characterized in that** said segments of pipe (6) in the part coming out from the reaction chamber have some holes (11) on the wall and some merlon-shaped engravings (7) at the ends.

7. Apparatus as claimed in claim 1, **characterized in that** said reaction chamber (1) has an inner diameter equal to 7.5-9.5 cm, said neck (2) of the reaction chamber has a height equal to 4.5 cm and said punch (5) has a height equal to 7.5 cm.

8. Apparatus as claimed in claim 1, **characterized in that** the various components are realized in a thermoplastic material including polyethylene, polypropylene and PVC.

## Patentansprüche

1. Einwegvorrichtung zur Produktion und Sofortabgabe von desinfizierenden Gasen, bestehend aus einer Reaktionskammer (1) in kugelförmiger Gestalt und mit einem zylindrischen Hals (2), aus einem zylindrischen einsetzbaren Behälter (3), der unten durch eine Membran (4) verschlossen ist und wie ein Stopfen in den Hals (2) eingesetzt wird, aus einem rohrförmigen Stempel (5), der in dem genannten einsetzbaren Behälter gleiten kann und am unteren Ende eine scharfe Kante hat, mit der er die Membran (4) durchstoßen kann, aus einem Deckel (12) für den genannten einsetzbaren Behälter, aus zwei abgestutzten Rohren (9), die so in die genannte Reaktionskammer hineinragen, dass ihre Lagen diametral entgegengesetzt sind und dass sie entlang einer gemeinsamen Achse verlaufen und in einem gewissen Abstand voneinander enden, aus zwei Rohrstücken (6), die mit den genannten abgestutzten Rohren jeweils ein durchgängiges Rohr bilden, das aus der genannten Reaktionskammer herausführt, und aus einem Bolzen (8) der sich vor der Verwendung der Vorrichtung luftdicht schließend in die zwei abgeschnittenen Rohre (9) einführen lässt, um das Eindringen von Luft in die Reaktionskammer zu verhindern.

2. Vorrichtung entsprechend Anspruch 1, **gekennzeichnet dadurch, dass** die Reaktionskammer (1) einen flachen Boden hat und dass der zylindrische Hals (2) sich oben an der Reaktionskammer (1) befindet.

3. Vorrichtung entsprechend Anspruch 1, **gekennzeichnet dadurch, dass** die scharfe Kante des Stempels (5) die Gestalt des Mundstücks einer Blockflöte hat.

4. Vorrichtung entsprechend Anspruch 1, **gekennzeichnet dadurch, dass** der Stempel (5) eine flache Basis mit einem Loch (14) hat.

5. Vorrichtung entsprechend Anspruch 1, **gekennzeichnet dadurch, dass** der Deckel (12) an seiner Grundfläche eine Einwölbung (13) hat, die vor der Verwendung der Vorrichtung das Loch (14) in der Basis des Stempels (5) verschließt.

6. Vorrichtung entsprechend Anspruch 1, **gekennzeichnet dadurch, dass** die Rohrstücke (6), die aus der Reaktionskammer herausführen, einige Löcher (11) in der Wand und einige zinnenförmige Aussparungen (7) an den Enden haben.

7. Vorrichtung entsprechend Anspruch 1, **gekennzeichnet dadurch, dass** die Reaktionskammer einen Innendurchmesser von 7,5-9,5 cm hat, der Hals (2) der Reaktionskammer 4,5 cm hoch ist und der Stempel (5) 7,5 cm hoch ist.

8. Vorrichtung entsprechend Anspruch 1, **gekennzeichnet dadurch, dass** die verschiedenen Komponenten aus einem thermoplastischen Werkstoff einschließlich Polyethylen, Polypropylen und PVC hergestellt sind.

## Revendications

1. Appareil à usage unique pour la production et la fourniture instantanée de gaz désinfectant, comprenant une chambre de réaction (1) ayant une forme sphérique et un goulot cylindrique (2), un bouchon creux (3) appliqué audit goulot (2) et ayant une forme cylindrique fermée à la partie inférieure par une membrane (4), un poinçon (5) ayant une structure tubulaire qui peut coulisser à l'intérieur dudit bouchon creux et se termine à la partie inférieure par un bord coupant pouvant percer ladite membrane (4), un couvercle (12) pour ledit bouchon creux, deux tubes tronqués (9) s'étendant vers l'intérieur dans ladite chambre de réaction et entrant dans la chambre de réaction à deux endroits diamétralement opposés et le long du même axe et se terminant à des endroits relativement distants l'un de l'autre, deux segments de tube (6) formant un tube continu avec lesdits tubes tronqués, qui sortent de ladite chambre de réaction, et une goupille (8) qui peut être insérée de façon étanche à l'air dans les deux tubes tronqués (9) avant l'utilisation de l'appareil afin d'empêcher l'air d'entrer dans la chambre de réaction.

2. Appareil selon la revendication 1, **caractérisé en ce que** ladite chambre de réaction (1) présente un fond plat, et ledit goulot cylindrique (2) est situé dans la partie supérieure de ladite chambre de réaction (1).

3. Appareil selon la revendication 1, **caractérisé en ce que** le bord coupant dudit poinçon (5) a la forme d'un bec de flûte.

4. Appareil selon la revendication 1, **caractérisé en ce que** ledit poinçon (5) comporte une base plane pourvue d'un trou (14).

5. Appareil selon la revendication 1, **caractérisé en ce que** ledit couvercle (12) comporte dans sa base une protubérance (13) qui ferme, avant l'utilisation, le trou (14) dudit poinçon (5).

6. Appareil selon la revendication 1, **caractérisé en ce que** lesdits segments de tube (6), dans la partie sortant de la chambre de réaction, comportent des trous (11) dans la paroi et des découpes crénelées (7) aux extrémités.

7. Appareil selon la revendication 1, **caractérisé en ce que** ladite chambre de réaction (1) a un diamètre intérieur égal à 7,5-9,5 cm, ledit goulot (2) de la chambre de réaction a une hauteur égale à 4,5 cm et ledit poinçon (5) a une hauteur égale à 7,5 cm.

8. Appareil selon la revendication 1, **caractérisé en ce que** les divers composants sont fabriqués en une matière thermoplastique incluant le polyéthylène, le polypropylène et le PVC.
